# EUROPEAN PATENT SPECIFICATION

(11) **EP 4 268 872 B1**
(45) Date of publication and mention of the grant of the patent: **04.06.2025**
(21) Application number: 23168188.3
(22) Date of filing: 17.04.2023
(51) Int. Cl.: A61M 11/00, A61M 16/06, A61M 15/00, A61M 16/10, A62B 18/00, A61M 16/00

(54) **MOBILE RESPIRATOR**
MOBILE ATEMSCHUTZVORRICHTUNG
RESPIRATEUR MOBILE

(30) Priority: 28.04.2022 TW 111204421 U
(43) Date of publication of application: 01.11.2023
(73) Proprietor: GrowTrend Biomedical Co., Ltd., Hsinchu City (TW)
(72) Inventor: OU, Pei-Yin, New Taipei City (TW); YU, Ching-Liang, Taoyuan City (TW); LIU, Yun-Yueh, Miaoli City, Miaoli County (TW); PAN, Neng Yu, New Taipei City (TW)
(74) Representative: Contadin, Giorgio

(56) References cited:
- EP-A2- 1 655 052
- WO-A1-2011/050059
- WO-A1-2016/162395
- WO-A1-2019/071296
- US-A- 4 019 508
- US-A1- 2005 103 343
- US-A1- 2007 089 743
- US-A1- 2015 320 954

## Description

### 1. Field of the Invention

The present disclosure relates to a respirator, and particularly to a mobile respirator.

### 2. Description of Related Art

Since nowadays air is seriously polluted, many patients having respiratory diseases may suffer allergy or other symptoms no matter indoors or outdoors. Thereby, many respirators and related products are provided on the market for patients to use during home sleep. The conventional respirator has a great volume and needs to be fixed on a platform and plugged in for use. Moreover, the conventional respirator is connected to an extra heating humidifier having a roughly same volume with the conventional respirator, which prevents the respiratory tract of the patient from dryness.

However, since the conventional respirator needs to be assembled and plugged in, the conventional respirator is limited for the patient to use only on the bed or in sleep. The conventional respirator is difficult to move with the patient. Although parts of the conventional respirators allow the patient to use in the car or taking public transportations, each one of these conventional respirators still has to operate with the electricity provided by a power supply on the transportation.

In addition, other conventional respirators are also disclosed in US2015/320954 A1, EP1655052 A2, US2007089743 A1, WO2019/071296 A1, WO2016/162395 A1, WO2011/050059 A1, US2005/103343 A1 and US4019508 A. In each one of the above-mentioned documents, a respirator having a battery for supplying power to the respiratory device is provided, allowing the respirator to be mobile.

To overcome the shortcomings of the conventional respirator, the present disclosure tends to provide a mobile respirator to mitigate or obviate the aforementioned problems. The invention is defined in the appended claims.

The main objective of the present disclosure is to provide a mobile respirator that has a respiratory device electrically connected to a power bank and is thus mobile.

The mobile respirator has a respiratory device, a pipe, a breathing component, and a power bank. The respiratory device has an inlet and an outlet fluidly communicating with an interior of the respiratory device. The pipe has two opposite ends, and one of the two opposite ends of the pipe is connected to the outlet of the respiratory device. The breathing component has a connecting tube connected to the other one of the two opposite ends of the pipe. The power bank is electrically connected to the respiratory device. The mobile respirator has an air supplier and a heat and moisture exchange filter having an inlet opening and an outlet opening; the inlet opening fluidly communicates with an interior of the heat and moisture exchange filter and is connected to the other one of the two opposite ends of the pipe; the outlet opening fluidly communicates with an interior of the heat and moisture exchange filter and connected to the connecting tube. The air supplier has a supplying pipe. The breathing component has a branch tube formed on the connecting tube, fluidly communicating with the connecting tube, and connected to an end of the supplying pipe away from the air supplier.

Other objectives, advantages and novel features of the disclosure will become more apparent from the following detailed description when taken in conjunction with the accompanying drawings.

In the drawings:
Fig. 1 is a front view of a preferred embodiment of a mobile respirator in accordance with the present invention omitting part of a pipe;
Fig. 2 is an operational view of the mobile respirator in Fig. 1; and
Fig. 3 is a front view of another embodiment of a mobile respirator in accordance with the present invention omitting part of the pipe.

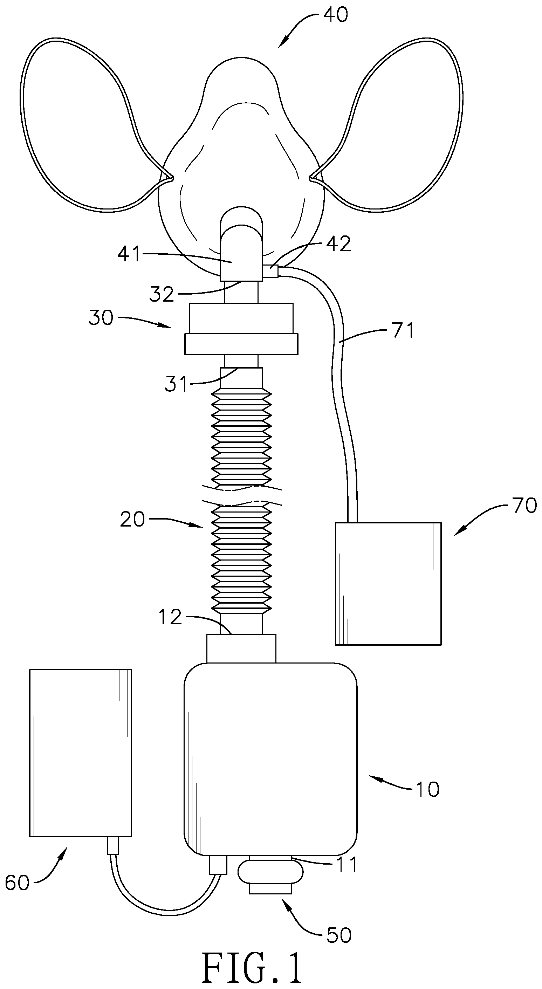

With reference to Fig. 1, a mobile respirator of a preferred embodiment in accordance with the present invention has a respiratory device 10, a pipe 20, a heat and moisture exchange filter (HMEF/FHME) 30, a breathing component 40, a filtering component 50, a power bank 60, and an air supplier 70.

With reference to Fig. 1, the respiratory device 10 has an inlet 11 and an outlet 12, and the inlet 11 and the outlet 12 fluidly communicate with an interior of the respiratory device 10. The respiratory device 10 is an auxiliary of an organ and is configured to generate positive pressure for assisting a patient in improving efficiency of gas exchange in the respiratory system of the patient. The respiratory device 10 has a portable construction such as a respiratory device in a 20 conventional micro respirator. The respiratory device 10 is conventional and is thus not described in detail.

With reference to Fig. 1, the pipe 20 has two opposite ends, and one of the two opposite ends of the pipe 20 is connected to the outlet 12 of the respiratory device 10 to make the pipe 20 fluidly communicate with the respiratory device 10. The pipe 20 can be a pipe having a fixed shape 25 or a flexible pipe such as a hosepipe. The pipe 20 is conventional and is thus not described in detail.

With reference to Fig. 1, the heat and moisture exchange filter (HMEF/FHME) 30 has an inlet opening 31 and an outlet opening 32, and the inlet opening 31 and the outlet opening 32 fluidly communicate with an interior of the heat and moisture exchange filter 30. The inlet opening 31 is connected to the other one of the two opposite ends of the pipe 20. The heat and moisture exchange filter 30 is a bacteria filter configured to heat and humidify air; moisture and heat exhaled by the patient are condensed and kept in the heat and moisture exchange filter 30 for heating and humidifying air later inhaled by the patient. Thereby, moisture of the air exhaled can be kept for increasing humidity of the air inhaled and prevent the air inhaled from dryness. The heat and moisture exchange filter 30 is a conventional component and is thus not described in detail. In other embodiments, the mobile respirator may omit the heat and moisture exchange filter 30, which is not limited by the preferred embodiment.

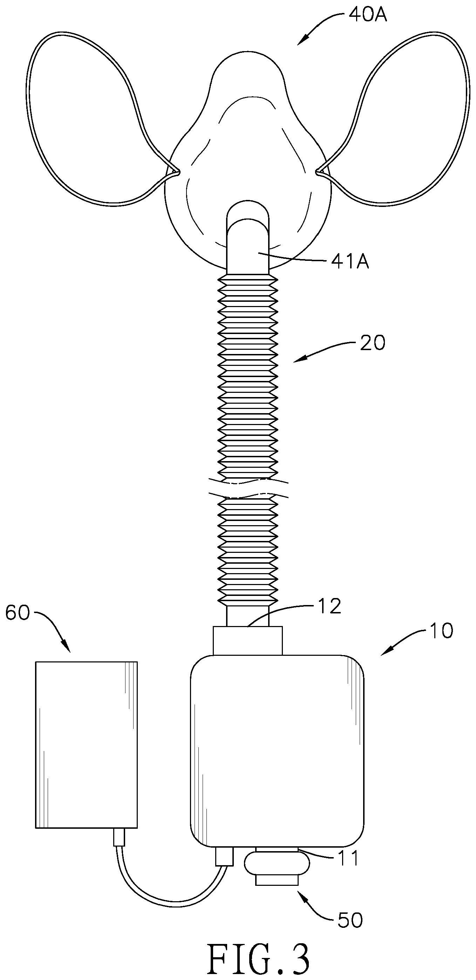

The breathing component 40 has a connecting tube 41 disposed on a side of the breathing component 40 and a branch tube 42. The branch tube 42 is formed on a surrounding wall of the connecting tube 41 and fluidly communicates with the connecting tube 41. The connecting tube 41 of the breathing component 40 is connected to the outlet opening 32 of the heat and moisture exchange filter 30. In the preferred embodiment, the breathing component 40 is a face mask, but in other embodiments, the breathing component 40 may be a nasal cannula or a nasal mask which allows air supplied from the respiratory device 10 to enter the nasal cavity of the patient. Configuration of the breathing component 40 is not limited to the preferred embodiment. Otherwise, in other embodiments, the breathing component 40 may omit the branch tube 42. With reference to Fig. 3, in a mobile respirator of an embodiment omitting the heat and moisture exchange filter 30, the connecting tube 41 of the breathing component 40 is directly connected to the pipe 20.

With reference to Fig. 1, the filtering component 50 is disposed to the inlet 11 of the respiratory device 10 for filtering pollutants such as dust. The filtering component 50 is conventional and is thus not described in detail. In other embodiment, the mobile respirator may omit the filtering component 50, which is not limited to the preferred embodiment.

With reference to Fig. 1, the power bank 60 is electrically connected to the respiratory device 10. The power bank 60 is conventional and is thus not described in detail.

With reference to Fig. 1, the air supplier 70 has a supplying pipe 71, and an end of the supplying pipe 71 away from the air supplier 70 is connected to the branch tube 42 of the breathing component 40. In the preferred embodiment, the air supplier 70 is a portable oxygen concentrator. In another embodiment, the air supplier 70 may supply another gas or atomized medication, and moreover, in other embodiments, the mobile respirator may omit the air supplier 70; the above configuration is not limited by the preferred embodiment.

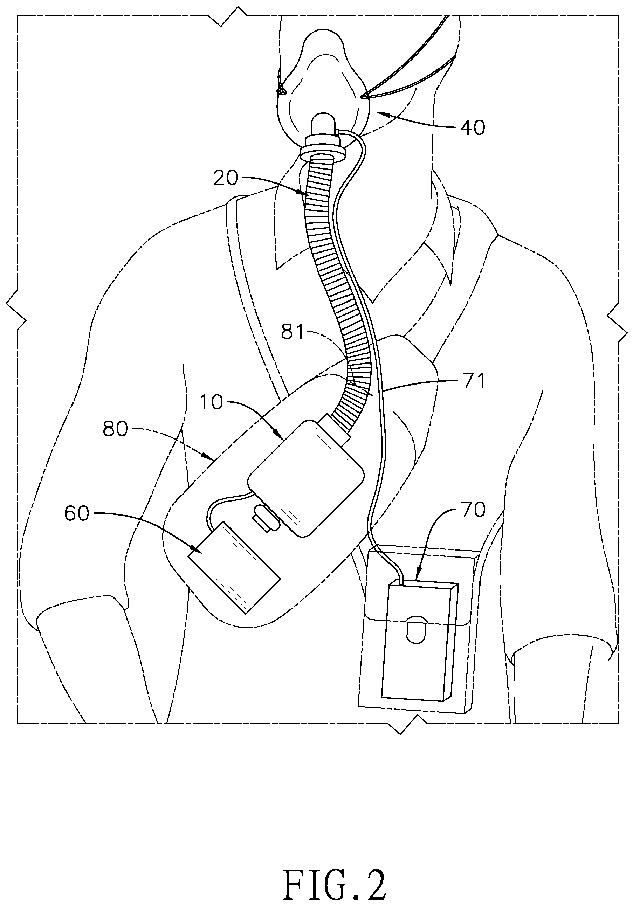

With reference to Fig. 2, in use of the mobile respirator of the preferred embodiment, the respiratory device 10, the power bank 60, and the air supplier 70 may be placed inside a backpack 80 having an opening 81 and multiple air openings (not shown in Figure); the pipe 20 and the supplying pipe 71 are outstretched from the opening 81 of the backpack 80. Thereby, a user can carry the backpack 80 and wear the breathing component 40, so the user may breathe with assistance of the mobile respirator while moving. The power bank 60 supplies electricity for the respiratory device 10, so the mobile respirator can be mobile outdoors and can solve the trouble of dyspnea while the user is exercising or staying outdoors. Compared to the conventional respirator only available at a fixed position, the mobile respirator of the present disclosure improves convenience in use.

Even though numerous characteristics and advantages of the present disclosure have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A mobile respirator comprising:
a respiratory device (10) having an inlet (11) and an outlet (12) fluidly communicating with an interior of the respiratory device (10);
a pipe (20) having two opposite ends, and one of the two opposite ends of the pipe (20) connected to the outlet (12) of the respiratory device (10);
a breathing component (40) having a connecting tube (41) connected to the other one of the two opposite ends of the pipe (20);
a power bank (60) electrically connected to the respiratory device (10);
an air supplier (70) having a supplying pipe (71); and
**characterized in that** the mobile respirator comprises
a heat and moisture exchange filter (30) having
an inlet opening (31) fluidly communicating with an interior of the heat and moisture exchange filter (30) and connected to the other one of the two opposite ends of the pipe (20); and
an outlet opening (32) fluidly communicating with the interior of the heat and moisture exchange filter (30) and connected to the connecting tube (41);
wherein the breathing component (40) has a branch tube (42) formed on the connecting tube (41), fluidly communicating with the connecting tube (41) and connected to an end of the supplying pipe (71) away from the air supplier (70).

2. The mobile respirator as claimed in claim 1, wherein the mobile respirator comprises a filtering component (50) disposed to the inlet (11) of the respiratory device (10).

3. The mobile respirator as claimed in claim 2, wherein the breathing component (40) is a face mask.

4. The mobile respirator as claimed in claim 2, wherein the breathing component (40) is a nasal cannula.

5. The mobile respirator as claimed in claim 2, wherein the breathing component (40) is a nasal mask.

## Patentansprüche

1. Mobile Atemschutzvorrichtung, umfassend:
eine Atmungsvorrichtung (10) mit einem Einlass (11) und einem Auslass (12),
die mit einem Inneren der Atmungsvorrichtung (10) in fluidtechnischer Verbindung stehen;
einen Schlauch (20) mit zwei gegenüberliegenden Enden und wobei eines der zwei gegenüberliegenden Enden des Schlauchs (20) mit dem Auslass (12) der Atmungsvorrichtung (10) verbunden ist;
eine Atmungskomponente (40) mit einem Verbindungsrohr (41), das mit dem anderen der zwei gegenüberliegenden Enden des Schlauchs (20) verbunden ist;
eine Powerbank (60), die elektrisch mit der Atmungsvorrichtung (10) verbunden ist;
eine Luftversorgungseinrichtung (70) mit einem Versorgungsschlauch (71); und
**dadurch gekennzeichnet, dass** die mobile Atemschutzvorrichtung umfasst einen Wärme- und Feuchtigkeitsaustauschfilter (30) mit
einer Einlassöffnung (31), die mit einem Inneren des Wärme- und
Feuchtigkeitsaustauschfilters (30) in fluidtechnischer Verbindung steht und mit dem anderen der zwei gegenüberliegenden Enden des Schlauchs (20) verbunden ist; und
einer Auslassöffnung (32), die mit dem Inneren des Wärme- und
Feuchtigkeitsaustauschfilters (30) in fluidtechnischer Verbindung steht und mit dem Verbindungsrohr (41) verbunden ist;
wobei die Atmungskomponente (40) ein an dem Verbindungsrohr (41) gebildetes Verzweigungsrohr (42) aufweist, das mit dem Verbindungsrohr (41) in fluidtechnischer Verbindung steht und mit einem von der Luftversorgungseinrichtung (70) entfernten Ende des Versorgungsschlauchs (71) verbunden ist.

2. Mobile Atemschutzvorrichtung nach Anspruch 1, wobei die mobile Atemschutzvorrichtung eine Filterungskomponente (50) umfasst, die an dem Einlass (11) der Atmungsvorrichtung (10) angeordnet ist.

3. Mobile Atemschutzvorrichtung nach Anspruch 2, wobei die Atmungskomponente (40) eine Atemmaske ist.

4. Mobile Atemschutzvorrichtung nach Anspruch 2, wobei die Atmungskomponente (40) eine Nasenkanüle ist.

5. Mobile Atemschutzvorrichtung nach Anspruch 2, wobei die Atmungskomponente (40) eine Nasenmaske ist.

## Revendications

1. Respirateur mobile comprenant :
un dispositif respiratoire (10) ayant une entrée (11) et une sortie (12) en communication fluidique avec un intérieur du dispositif respiratoire (10) ;
un tuyau (20) ayant deux extrémités opposées, et une des deux extrémités opposées du tuyau (20) reliée à la sortie (12) du dispositif respiratoire (10) ;
un composant respiratoire (40) ayant un tube de raccordement (41) relié à l'autre des deux extrémités opposées du tuyau (20) ;
une banque d'alimentation (60) reliée électriquement au dispositif respiratoire (10) ;
un fournisseur d'air (70) ayant un tuyau d'alimentation (71) ; et
**caractérisé en ce que** le respirateur mobile comprend
un filtre d'échange de chaleur et d'humidité (30) ayant
une ouverture d'entrée (31) en communication fluidique avec un intérieur du filtre d'échange de chaleur et d'humidité (30) et reliée à l'autre des deux extrémités opposées du tuyau (20) ; et
une ouverture de sortie (32) en communication fluidique avec l'intérieur du filtre d'échange de chaleur et d'humidité (30) et reliée au tube de raccordement (41) ;
dans lequel le composant respiratoire (40) a un tube de ramification (42) formé sur le tube de raccordement (41), en communication fluidique avec le tube de raccordement (41) et relié à une extrémité du tuyau d'alimentation (71) éloignée du fournisseur d'air (70).

2. Respirateur mobile selon la revendication 1, dans lequel le respirateur mobile comprend un composant filtrant (50) disposé sur l'entrée (11) du dispositif respiratoire (10).

3. Respirateur mobile selon la revendication 2, dans lequel le composant respiratoire (40) est un masque facial.

4. Respirateur mobile selon la revendication 2, dans lequel le composant respiratoire (40) est une canule nasale.

5. Respirateur mobile selon la revendication 2, dans lequel le composant respiratoire (40) est un masque nasal.
